# EUROPÄISCHE PATENTSCHRIFT

(11) **EP 2 856 939 B1**
(45) Veröffentlichungstag und Bekanntmachung des Hinweises auf die Patenterteilung: **26.07.2017**
(21) Anmeldenummer: 14193694.8
(22) Anmeldetag: 03.09.2004
(51) Int. Cl.: A61B 5/087

(54) **Verfahren zur Observation schlafbezogener Atmungsstörungen**
Method for observing sleep-related breathing disorders
Procédé d'observation de troubles respiratoires liés au sommeil

(30) Priorität: 03.09.2003 DE 10340654
(43) Veröffentlichungstag der Anmeldung: 08.04.2015
(62) Teilanmeldung aus: 04764809.2
(73) Patentinhaber: ResMed R&D Germany GmbH, 82152 Martinsried (DE)
(72) Erfinder: Madaus, Stefan, 82166 Gräfelfing (DE); Graf von Stauffenberg, Caspar, 82131 Gauting (DE); Vögele, Harald, 82131 Gauting (DE); Heidmann, Dieter, Castle Hill, NSW 2154 (AU); Klaus, Dieter, 79689 Maulburg (DE)
(74) Vertreter: Vossius & Partner Patentanwälte Rechtsanwälte mbB

(56) Entgegenhaltungen:
- WO-A-93/09834
- WO-A1-00/33735
- WO-A1-02/00283
- WO-A2-02/082997
- DE-A1- 10 164 445

## Beschreibung

Die Erfindung betrifft ein Verfahren zur Erfassung und Auswertung eines hinsichtlich der Atmung einer schlafenden Person indikativen Messsignals im Zusammenhang mit der Observation schlafbezogener Atmungsstörungen.

Zur Untersuchung schlafbezogener Atmungsstörungen sind sog. Polysomnographiegeräte bekannt, die üblicherweise über mehrere Messkanäle zur Erfassung verschiedener Messsignale für physiologische Zustandsgrößen eines Patienten, verfügen. Wie aus der auf die Anmelderin zurückgehenden Patentschrift DE 101 64 445 A1 hervorgeht, ist es bekannt, zur Diagnose eines Patienten im Hinblick auf dessen Atmungseigenschaften während einer Schlafphase EKG- und Blutdrucksignale zu erfassen und diese gemeinsam mit Signalen, die die Atmungstätigkeit des Patienten beschreiben, aufzuzeichnen. Jene die Atmungstätigkeit des Patienten beschreibenden Signale können über sog. Thermistoren oder auch über Pneumotachographen generiert werden. Die erfassten Signale können in zeitlicher Zuordnung zueinander visualisiert und im Rahmen einer fachärztlichen Betrachtung ausgewertet werden. Auf Grundlage der fachärztlichen Auswertung kann festgestellt werden, ob etwaigen Atmungsstörungen durch Zufuhr des Atemgases auf einem erhöhten Druckniveau (CPAP-Therapie) vorgebeugt werden kann. Geeignete Therapieparameter können ebenfalls im Rahmen der fachärztlichen Auswertung ermittelt werden.

Bei der Untersuchung von Personen mit Anzeichen auf schlafbezogene Atmungsstörungen führt die Betrachtung der generierten Messsignale in der Praxis unter Umständen zu unterschiedlichen Befunden insbesondere hinsichtlich Art- und Schweregrad obstruktionsrelevanter Beeinträchtigungen der Atemwege sowie hinsichtlich der Bewertung des allgemeinen physiologischen Zustands des Patienten. Bei unzureichender Bewertung eines Krankheifisbildes besteht das Problem, dass ein ggf. erforderlicher. Therapiebedarf nicht erkannt, oder für eine Therapie Rahmenbedingungen gewählt werden, die den tatsächlichen physiologischen Bedürfnissen nicht hinreichend Rechnung tragen, oder zumindest den Therapiekomfort einschränken.

Der Entschluss, sich zur Untersuchung auf etwaige schlafbezogene Atmungsstörungen in ein Schlaflabor zu begeben, wird vom Betroffenen häufig erst getroffen, wenn Folgeerscheinungen des Krankheitsbildes OSA das Befinden desselben bereits erheblich belasten. Die durch OSA verursachte Belastung des Betroffenen erschwert im fortgeschrittenen Stadium der Erkrankung die präzise Diagnose derselben.

Der Erfindung liegt die Aufgabe zugrunde, Lösungen bereitzustellen, die es ermöglichen, eine besonders aussagefähige Untersuchung hinsichtlich des Auftretens schlafbezogener Atmungsstörungen, insbesondere in der gewohnten Umgebung des Betroffenen durchzuführen.

Gemäß der vorliegenden Erfindung wird diese Aufgabe erfindungsgemäß durch ein Verfahren gemäß Anspruch 1 gelöst. Dadurch wird es auf vorteilhafte Weise möglich, mittels einer zur Selbstapplikation geeigneten Erfassungseinrichtung im häuslichen Bereich, d.h. in der gewohnten Umgebung des Betroffenen hinsichtlich der Atmung während einer Schlafphase charakteristische Merkmale in einer Weise zu erfassen, die es gestattet, den physiologischen Zustand des Betroffenen mit besonders hoher Aussagekräftigkeit zu bestimmen. Auf Grundlage einer standardisierten, rechnerbasierten Auswertung der Messergebnisse kann nachfolgend beurteilt werden, ob - und ggf. mit welchem Schweregrad das Krankheitsbild OSA vorliegt und ob eine tiefergehende Untersuchung durch ein Schlaflabor zu empfehlen ist.

Die Datenverarbeitungseinrichtung, oder ein dieser vorgelagerter Signalübertragungsschaltkreis ist vorzugsweise derart konfiguriert, dass eine Prüfung ermöglicht ist, ob das jeweils erfasste Atemstromsignal vorgegebene Signalqualitätskriterien erfüllt. Für den Fall, dass das anliegende Signal bestimmte Kriterien nicht erfüllt, ist es möglich die Signalaufzeichnung zu unterdrücken, oder auch Aufzeichnungen vorzunehmen, aus welchen die zeitliche Lage von als unzulässig klassifizierten Signalen hervorgeht.

Die Datenverarbeitungseinrichtung ist vorzugsweise derart ausgebildet, dass diese Zugriff zu einer Zeitgebereinrichtung hat, und dass die hinsichtlich des jeweiligen Atemgasstroms indikativen Daten mit einer Zeitinformation verknüpft aufgezeichnet werden.

In Verbindung mit jener Datenverarbeitungseinrichtung ist vorzugsweise ein Datenkompressionssystem verwirklicht, durch welches die erfassten, sich zeitlich ändernden Signale komprimiert aufgezeichnet werden können.

Die Datenverarbeitungseinrichtung ist weiterhin vorzugsweise derart konfiguriert, dass der Aufzeichnungsvorgang durch einen anwenderseitig veranlassten Schaltimpuls ausgelöst wird. Es ist möglich, den Schaltimpuls in Abhängigkeit davon zu generieren, ob ein Schalttaster über eine vorgegebene Mindestdauer von z.B. 3 Sekunden betätigt wurde.

Die Datenverarbeitungseinrichtung kann derart ausgebildet sein, dass diese die Datenspeicherung veranlasst, wenn die anliegenden Atemstromsignale ein bestimmtes Kriterium z.B. ein vorgegebenes Periodizitätskriterium erfüllen.

Vorzugsweise umfasst ein erfindungsgemäß verwendbares Erfassungsgerät einen ersten Druckmessanschluss an welchen eine Messkanüle, ein Messkanülenpaar, oder ein Messkanülenbündel anschließbar ist. Die erste Messkanüle kann mit einer Nasaldruckmessbrilleneinrichtung verbunden sein, zum Abgriff eines Staudrucksignals aus dem, aus den Nasenöffnungen abströmenden Atemgasstrom. Die zweite Messkanüle kann derart an den Anwender herangeführt werden, dass über diese ein hinsichtlich des über den Mund des Anwenders ggf. fließenden Gasstroms indikatives Signal erhebbar ist.

Es ist möglich, das Erfassungsgerät mit einem zweiten ebenfalls der Erfassung der nasalen Atmung dienenden Druckmessanschluss auszustatten, zur Erfassung eines zweiten Nasalatemgasstromsignals. Durch die Möglichkeit der Erfassung von zwei Druckmessignalen wird es möglich, das Erfassungsgerät derart zu betreiben, dass dieses für die linke und rechte Nasenöffnung jeweils indikative Atemstromsignale erfassen kann.

Vorzugsweise umfasst das Erfassungsgerät eine Einrichtung zur Erfassung der Thoraxweitung. Die Einrichtung zur Erfassung der Thoraxweitung kann ein Bandelement umfassen, das um den Thoraxbereich des Anwenders herumführbar ist. Es ist möglich, dieses Bandelement derart auszugestalten, dass aus diesem ein hinsichtlich der Banddehnung, oder Bandbelastung indikatives Signal ableitbar ist (z.B. Änderung des elektrischen Widerstands eines eingebetteten Leiters). Es ist auch möglich, an dem Erfassungsgerät Vorkehrungen zu treffen, durch welche die Bandbelastung erfasst werden kann. Insbesondere ist es möglich, an dem Erfassungsgerät eine Schlaufeneinrichtung vorzusehen, durch welche die Bandweitung, oder Bandkräfte erfasst werden können. Es ist auch möglich, an dem Erfassungsgerät Druck- oder Krafterfassungsstrukturen vorzusehen, durch welche die durch ein über diese Krafterfassungsstrukturen geführtes Band aufgebrachten Kräfte erfasst werden können. Das Band zur Erfassung der Thoraxweitung kann zugleich der Befestigung des Erfassungsgeräts am Anwender dienen.

Vorzugsweise ist der Aufzeichnungsvorgang durch ein anwenderseitig veranlassbares Schaltsignal beendbar. Dieses Schaltsignal kann insbesondere über den vorangehend zum Einschalten des Geräts verwendeten Schalter, insbesondere Druckknopf, generiert werden. Es ist weiterhin auch möglich, das Erfassungsgerät derart zu konfigurieren, dass der Aufzeichnungsvorgang durch Erfüllung eines Zeitkriteriums beendbar ist. Insbesondere ist es möglich, den Aufzeichnungsvorgang zu beenden, wenn dieser eine vorgegebene Aufzeichnungsdauer von z.B. 9,5 h erreicht hat.

Weiterhin ist es möglich, den Aufzeichnungsvorgang schaltungsgesteuert zu beenden, wenn das anliegende Atemstromsignal über ein bestimmtes Abschaltzeitfenster ein bestimmtes Abschaltkriterium erfüllt. Es ist möglich, die Aufzeichnungskapazität auf eine bestimmte Anzahl von Aufzeichnungen, insbesondere auf zwei Aufzeichnungen zu begrenzen.

Gemäß einem besonderen Aspekt ist das Erfassungsgerät mit einer Schnittstelleneinrichtung versehen, zur Übertragung der aufgezeichneten Daten auf ein externes Auswertungssystem. Diese Schnittstelleneinrichtung ist vorzugsweise als USB-Schnittstelle oder auch als Infrarot-Schnittstelle ausgebildet.

In besonders vorteilhafter Weise ist es auch möglich, das Erfassungsgerät derart zu gestalten, dass die Speichereinrichtung austauschbar in dem Erfassungsgerät aufgenommen ist. Die Speichereinrichtung kann hierzu als Karte oder insbesondere USB-Flashstick ausgebildet sein. Es ist möglich, durch einen vorab auf der Speichereinrichtung veranlassten Eintrag personenspezifische Daten auf dem Speichermedium aufzuzeichnen. Auf Grundlage dieser Vorab-Aufzeichnungen ist es möglich, eine Vorkonfiguration des Erfassungsgerätes vorzunehmen, oder eine zutreffende Zuordnung der erfasten Daten zu dem Anwender sicherzustellen.

Die Konfiguration der Datenverarbeitungseinrichtung ist vorzugsweise durch ein Datenverarbeitungsprogramm festgelegt, wobei dieses Datenverarbeitungsprogramm vorzugsweise veränderbar oder wechselbar ist. Die Abbildung des Datenverarbeitungsprogramms im Bereich des Erfassungsgerätes kann über die vorgenannten Schnittstelleneinrichtungen, zusätzliche Schnittstelleneinrichtungen, oder auch das Speichermedium erfolgen.

An dem Erfassungsgerät sind neben vorzugsweise intuitiv leicht zu bedienenden Schaltereinrichtung auch Anzeigeeinrichtungen vorgesehen, zur Anzeige der Betriebsbereitschaft oder des Funktionszustands des Erfassungsgerätes. Es ist möglich, die Aufzeichnungsbereitschaft des Erfassungsgerätes durch periodisches Aufblinken einer Signaldiode zu signalisieren.

Das Erfassungsgerät umfasst vorzugsweise eine Spannungsversorgungseinrichtung, die beispielsweise durch eine Batterieeinrichtung gebildet sein kann. Vorzugsweise ist die Batterieeinrichtung möglichst kompakt ausgebildet, so dass das Erfassungsgerät flach und kleinbauend ausgeführt werden kann und nur ein geringes Eigengewicht aufweist.

Die Datenverarbeitungseinrichtung ist vorzugsweise mit einer Kalibriereinrichtung gekoppelt, zur Kalibrierung des Atemstromsignals. Die Kallibriereinrichtung kann derart ausgebildet sein, dass diese eine selbsttätige Anpassung des Systems an die erfassten Signalpegel vornimmt.

Gemäß einem besonderen Aspekt ist das Erfassungsgerät derart aufgebaut, dass dieses eine Strukturkomponente aufweist, die zu einem Wiedergabegerät kompatibel ist, das in seinem Aufbau einem Game-Boy entspricht.

Es ist möglich, das Erfassungsgerät derart auszugestalten, dass zumindest ein Abschnitt desselben in einen Einschubschacht eines Game-Boy einführbar ist.

Gemäß einem besonderen Aspekt ist das Erfassungsgerät derart ausgebildet, dass dieses ein Basismodul und ein mit diesem koppelbares Aufzeichnungstransfermodul umfasst.

Vorzugsweise ist das Aufzeichnungsmodul als jene Game-Boy kompatible Struktur ausgebildet. Hierdurch wird es möglich, die aufgezeichneten Daten über eine intuitiv leicht verständliche Benutzeroberfläche über ein handelsübliches Nutzerendgerät zu visualisieren und ggf. im Hinblick auf ausgewählte Eigenschaften zu verarbeiten.

Über das Basismodul erfolgt vorzugsweise die Spannungsversorgung, sowie die Konvertierung des Drucksignales. Das Basismodul umfasst hierzu vorzugsweise einen Batterieschacht und einen Drucksensor.

Das Aufzeichnungsmodul umfasst vorzugsweise eine Datenverarbeitungseinrichtung die derart konfiguriert ist, dass diese eine Aufzeichnung von hinsichtlich des zeitlichen Verlaufs der Atmung indikativen Signalen auf einer Speichereinrichtung veranlasst. Das Aufzeichnungsmodul kann mit einer Schnittstelleneinrichtung versehen sein, zum Auslesen der aufgezeichneten Daten. Es ist möglich, die Speichereinrichtung lösbar mit dem Aufzeichnungsmodul zu koppeln, so dass es möglich wird, die Speichereinrichtung von dem Aufzeichnungsmodul zu trennen und einem anderweitigen System zur weiteren Auswertung und Visualisierung zuzuführen.

Die Erfassung des Atmungssignales kann alternativ zu einer Erfassung unter Verwendung einer Nasalkanüleneinrichtung auch über anderweitige Messmittel erfolgen.

Die eingangs angegebene Aufgabe wird erfindungsgemäß auch gelöst durch ein Verfahren zur Bereitstellung eines hinsichtlich des physiologischen Zustands indikativen Auswertungsresultats auf der Grundlage von Messsignalen die mit der Atmung einer Person im Zusammenhang stehen, wobei aus den genannten Messsignalen unter Heranziehung mehrerer, Auswertungssysteme, Auswertungsmerkmale generiert werden und im Rahmen eines hierauf basierenden Resultat-Generierungsschrittes wenigstens ein Auswertungsresultat generiert wird, indem die Auswertungsmerkmale einer verknüpfenden Betrachtung unterzogen werden, wobei die Messsignale im Rahmen einer der Auswertung vorangehenden Signalerfassungsphase ambulant durch ein seitens des Betroffenen applizierbares Erfassungsgerät erfasst werden.

Dadurch wird es auf vorteilhafte Weise möglich, im Rahmen einer ca. 6 bis 8 Stunden andauernden anwenderseitig ambulant durchgeführten Signalerhebung eine Datenmenge zu schaffen, aus welcher Auswertungsmerkmale generiert werden können, aus welchen mit hoher Aussagefähigkeit in einer standardisiert wiederholbaren Weise Auswertungsresultate gewonnen werden können, die in vorteilhafter Weise einer nachfolgenden Diagnose zugrundegelegt werden können und damit eine standardisierten Vorabbewertung ermöglichen.

Die verknüpfende Betrachtung der für die einzelnen Atemzüge ermittelten Atemzugseigenschaften kann sich über ein Zeitfenster erstrecken das beispielsweise eine vorgegebene z.B. 30 Atemzüge oder adaptiv optimierte Anzahl von Atemzügen überblickt. Insbesondere für die Beurteilung des physiologischen Zustands des Anwenders, beispielsweise als Grundlage für eine ärztliche Diagnose, ist es auch möglich, bestimmte Verknüpfungsoperationen, für schlafphasenbezogene Perioden, ausgewählte Zeitabschnitte oder auch die gesamte Messung umfassende Zeitfenster durchzuführen. Gemäß einer besonders bevorzugten Ausführungsform der Erfindung werden auf Grundlage von Verknüpfungsoperationen Rohdaten und/oder Zwischenergebnisse selektiert die eine besonders zuverlässige Generierung von charakteristischen Kennwerten insbes. Indizes ermöglichen.

Gemäß einer besonders bevorzugten Ausführungsform der Erfindung erfolgt auf der Grundlage der verknüpfenden Betrachtung eine physiologische Typisierung des gegebenenfalls vorhandenen Krankheitsbildes der untersuchten Person.

Gemäß einer besonders bevorzugten Ausführungsform der Erfindung werden die Auswertungsmerkmale auf Grundlage von Korrelationskriterien insbesondere statistischen Auswertungssystemen generiert, durch welche beispielsweise Gemeinsamkeiten mit vorangegangenen Atemzügen, oder vorzugsweise adaptiv optimierten Bezugskriterien z.B. zu Referenzatemzügen bewertet werden. Die Korrelationskriterien können insbesondere auch auf die erste und/oder zweite Ableitung des erfassten Atemgasstromes angewendet werden. Eine Generierung der für den jeweiligen Atemzug charakteristischen Merkmale kann unter Heranziehung statistischer Methoden erfolgen. Auch die verknüpfende Betrachtung der für jeden Atemzug ermittelten Eigenschaften kann unter Heranziehung statistischer Methoden erfolgen.

Auf Grundlage der für jeden Atemzug oder auch bestimmte Atemzugsfolgen generierten Auswertungsmerkmale kann sukzessive ein Merkmalsarray aufgefüllt werden, wobei dieses Merkmalsfeld zumindest für ausgewählte Auswertungsmerkmale ein Zeitfenster beschreibt, das wenigstens so groß ist, wie das kleinste, zur verknüpfenden Betrachtung der Auswertungsmerkmale vorgesehene Verknüpfungszeitfenster.

Die Auswertungsmerkmale werden gemäß einer besonders bevorzugten Ausführungsform der Erfindung derart generiert, dass sich unter diesen z.B. Auswertungsmerkmale befinden, die Angaben zur Dauer eines Atemzuges und/oder z.B. hinsichtlich als normal anzusehender Atemzüge charakteristische Angaben enthalten. Auf Grundlage dieser Auswertungsmerkmale wird es möglich, die zeitliche Länge von Perioden mit normaler Atmung, im Rahmen der verknüpfenden Betrachtung zu bestimmen.

Weiterhin werden die Auswertungsmerkmale in vorteilhafter Weise auch derart generiert, dass diese das Auftreten etwaiger Flusslimationserscheinungen in einzelnen Atemzügen vorzugsweise auch bestimmte, hinsichtlich der Flusslimitation repräsentative Angaben enthalten. Auf Grundlage einer verknüpfenden Betrachtung der für derartige flusslimitierte Atemzüge erfassten Auswertungsmerkmale wird es möglich, die zeitliche Dauer bestimmter Eigenschaften von zumindest teilweise flusslimitierten Atmungssequenzen zu beschreiben.

Für Perioden in denen keine Atmungstätigkeit erfasst wird, können ebenfalls Auswertungsmerkmale generiert werden anhand welcher die Phasenlänge etwaiger Apnoesequenzen und/oder auch für Eigenschaften dieser Apnoephase charakteristische Merkmale im Rahmen der verknüpfenden Betrachtung generiert werden können. Diese Auswertungsmerkmale enthalten vorzugsweise Informationen hinsichtlich der Art der Apnoephasen z.B. darüber, ob die Apnoephase als zentrale, als obstruktive oder als Kombination (gemischte Apnoephase) einzustufen ist.

Derartige Auswertungsmerkmale werden gemäß einer besonders bevorzugten Ausführungsform der Erfindung auch für Schnarchphasen, für Phasen mit Cheyne-Stokes Atmung und Hypoventilationphasen generiert.

In den Auswertungsmerkmalen sind vorzugsweise auch Angaben bzw. Informationen enthalten aus welchen die Körperposition, die Kopfposition und vorzugsweise auch der Halsverdrehungsgrad des Anwender ableitbar ist. Bereits in den Auswertungsmerkmalen können schlafphasenindikative Angaben enthalten sein.

Die generierten Auswertungsmerkmale werden vorzugsweise unter Zuordnung zu dem erfassten Atemzug bzw. unter Berücksichtigung ihrer zeitlichen Lage gespeichert. D.h. die generierten Auswertungsmerkmale sind einem definierten Zeitfenster - im Falle normaler Atmung dem jeweiligen Atemzug zuordbar.

Weiterhin ist es möglich, im Rahmen der verknüpfenden Betrachtung, einen Schnarchindex zu generieren.

Weiterhin ist es möglich im Rahmen der verknüpfenden Betrachtung einen Schlafphasenindex zu generieren. Es ist in Verbindung mit der Atemphasenbetrachtung möglich zwischen inspiratorischem (obstruktionsrelevantem) und expiratorischem (minderrelevantem) Schnarchen zu unterscheiden. Weiterhin ist es möglich im Rahmen der verknüpfenden Betrachtung einen periodischen Atmungsindex zu generieren. Weiterhin ist es möglich im Rahmen der verknüpfenden Betrachtung einen Atemvolumenindex zu generieren.

Die Messung des Atemgasstromes kann bei Umgebungsdruck oder auch unter definiert verändertem Atemgasdruck erfolgen.

Zumindest ein Teil der Auswertungsmerkmale wird vorzugsweise unter Berücksichtigung der ersten oder zweiten Ableitung des zeitlichen Verlaufs des Atemgasstromes generiert.

Gemäß einem weiteren Aspekt der vorliegenden Erfindung wird die eingangs angegebene Aufgabe auch gelöst, durch eine Vorrichtung zur Durchführung des vorangehend beschriebenen Verfahrens, wobei diese Vorrichtung eine Messsignaleingabeeinrichtung und eine Rechnereinrichtung zur Bereitstellung mehrerer Auswertungssysteme umfasst, wobei aus den genannten Messsignalen durch die Auswertungsysteme, Auswertungsmerkmale generiert werden und im Rahmen eines hierauf basierenden Resultat-Generierungsschrittes wenigstens ein Auswertungsresultat generiert wird indem die Rechnereinrichtung derart konfiguriert ist, dass diese die Auswertungsmerkmale einer verknüpfenden Betrachtung unterzieht.

Im Rahmen der Erfassung der Atmungstätigkeit des Anwender auf Grundlage von hinsichtlich des Atemgasvolumenstromes indikativen Daten ist es möglich, einzelne Atemzüge als solche zu erkennen. Anfang und Ende der In- und Expirationsphasen des Atemzuges können beispielsweise in Verbindung mit einer Untersuchung der ersten und zweiten Ableitung des Atemgasstromsignals sowie auch unter Berücksichtigung des möglichen Atemzugvolumens bestimmt werden. Auf Grundlage dieser Auswertungsergebnisse kann die Zeitdauer der Atemzugsphasen das tatsächliche Volumen des Atemzugs und das Atemmuster bestimmt werden.

Durch eine statistische Analyse der Eigenschaften mehrerer abfolgender Atemzüge, kann der physiologische Zustand der untersuchten Person weiter bestimmt werden. Auf Grundlage der Extraktion von Merkmalen für jeden einzelnen Atemzug kann eine Rohdatenreduzierung erreicht werden. Aus der statistischen Auswertung der Eigenschaften mehrerer abfolgender Atemzüge kann zwischen obstruktionsrelevantem Schnarchen und obstruktionsunrelevantem Schnarchen unterschieden werden. Eine Typisierung von Oszillationseigenschaften in Verbindung mit Schnarchereignissen kann hierbei unter Verzicht auf eine Mikrophoneinrichtung erfolgen.

Das Auftreten etwaiger schnarchbedingter Oszillationen kann anhand des zeitlichen Verlaufs des Atemsignals erfasst werden. So ist es beispielsweise möglich, durch Schnarchen verursachte Druckoszillationen aus, durch entsprechende Drucksensoreinrichtungen generierten Signalen zu extrahieren. Insbesondere auf Grundlage einer Frequenz- und Amplitudenanalyse z.B. Fast Fourier-Analyse ist es möglich, Schnarchereignisse hinsichtlich ihres Entstehungsortes (Gaumensegel, Kehlkopf...) zu klassifizieren.

Auf Grundlage der verknüpfenden Betrachtung der Auswertungsmerkmale können insbesondere die folgenden obstruktiven Atmungsstörungen (OSA) erkannt werden:
Apnoe, Hypopnoe, flusslimitierte Atmung, sowie stabile und instabile Atmung.

Eine Atmungsstörung wird als Apnoeereignis klassifiziert, wenn ein Atmungsstillstand erfasst wird, dessen Länge eine vorbestimmte Zeitdauer von beispielsweise 10 Sekunden übersteigt.

Ein Hypopnoeereignis kann dann als vorliegend angesehen werden, wenn nach drei beispielsweise als normal klassifizierten Atemzügen, mindestens zwei sowie maximal drei größere Atemzüge erkannt werden. Als weiteres Kriterium hierfür kann das inspiratorisches Differenzvolumen der betrachteten Atemzüge herangezogen werden.

In dem jeweils untersuchten Atemzug kann eine Flusslimitation erkannt werden, wenn der Atemgasstrom während der Inspirationsphase bestimmte Plateauzonen oder mehrere Maxima aufweist.

Etwaige in einem Drucksignal auftretende hochfrequente Oszillationen können in Verbindung mit dem Atemflusssignal als in- oder expiratorisches Schnarchen klasssifiziert werden. Die hinsichtlich des Auftretens von Schnarchen generierten Auswertungsmerkmale können in die zur Generierung der Auswertungsresultate vorgesehene verknüpfende Betrachtung Eingang finden.

Die erfindungsgemäße Erfassung und Auswertung der hinsichtlich des Atemgasstromes indikativen Signale kann Informationen zur Beschreibung und Visualisierung des physiologischen Zustands einer Person insbesondere im Hinblick auf eine mit schlafbezogenen Atmungsstörungen in Verbindung stehende Erkrankung geben. Die erfindungsgemäße Signalerfassung und Auswertung kann zur Konfiguration von Beatmungsgeräten herangezogen werden.

Gemäß einem besonderen Aspekt der Erfindung werden wenigstens zwei der nachfolgenden Angaben kombiniert angewendet:
- Der Grad der statistischen Sicherheit der ermittelten Beurteilungs- oder Klassifizierungsergebnisse wird ermittelt.
- Für jeden Atemzug werden atemzugsspezifische Merkmale nach Maßgabe definierter Analyseprozeduren ermittelt.
- Die Analyseprozeduren berücksichtigen insbesondere den Inspirationsvorgang, den Expirationsvorgang, den Übergang zwischen den genannten Vorgängen, Kurveneigenschaften des Atemgasstromverlaufs innerhalb jedes Atemzyklus, Kombinationsbetrachtungen von Merkmalen des Atemgasstromverlaufes innerhalb eines Atemzuges.

- Die Gemeinsamkeiten von Atemzügen werden ermittelt.
- Unterschiede oder zeitliche Änderungen von Atemzugsmerkmalen werden ermittelt und bei der Beurteilung des physiologischen Zustands des Anwender berücksichtigt.
- Aus einer mehrfach verknüpften Betrachtung von Einzelmerkmalen werden Auswertungsresultate generiert die standardisiert parametrisch einen physiologischen Zustand oder physiologische Eigenschaften beschreiben.

Weitere Einzelheiten und Merkmale der Erfindung ergeben sich aus der nachfolgenden Beschreibung in Verbindung mit der Zeichnung. Es zeigen:
- **Fig.1a**: eine perspektivische Ansicht einer ersten Ausführungsform eines erfindungsgemäß verwendbaren Erfassungsgerätes;
- **Fig.1b**: eine Skizze zur Illustration einer Nasalkanüleneinrichtung zur Erfassung des Atemgasstromes durch Staudruckabgriff im Bereich der Nasenöffnungen;
- **Fig.1c**: eine Skizze zur Illustration einer Kanüleneinrichtung zur Erfassung des Atemgasstromes durch Druckabgriff im Innenbereich einer Einwegfiltermaske;
- **Fig.1d**: eine Skizze zur Illustration einer Applikationsweise des Erfassungsgerätes am Anwender unter Verwendung eines den Thorax umgreifenden Bandes;
- **Fig.1e**: eine Skizze zur Illustration einer zweiten Ausführungsform eines Erfassungsgerätes mit mehreren Anschlussabschnitten zum Anschluss von Druckmesskanülen zur separaten Erfassung nasaler und oraler Atmung;
- **Fig.1f**: eine Skizze zur Erläuterung eines Konzeptes zur Überwachung der Atmung durch Abgriff von, für den Atemgasstrom durch die linke Nasenöffnung, die rechte Nasenöffnung sowie den Mund des Anwenders indikativen Signalen
- **Fig**.**1g**: eine Skizze zur Erläuterung Nasenbrillenanordnung mit integrierter Kanüle zur Erfassung der Mundatmung;
- **Fig.1h**: eine Skizze zur Erläuterung eines Konzeptes zur Überwachung der Atmung durch Abgriff unter Verwendung einer Vollgesichtsmaske;
- **Fig**.**1i**: eine Skizze zur Erläuterung einer zur Applikation auf der Nase eines Anwenders vorgesehenen Abgriffseinrichtung;
- **Fig.1j**: eine Skizze zur Erläuterung einer für eine Abgriffseinrichtung nach Figur 1i geeigneten Strömungsweggestaltung;
- **Fig.1k**: eine Skizze zur Erläuterung einer zur nasalen Applikation vorgesehenen, die Nasenöffnungen eines Anwenders abdeckenden Abgriffseinrichtung;
- **Fig**.**1l**: eine Skizze zur Erläuterung einer zum Ansatz an den Nasenöffnungsbereich vorgesehenen Abgriffseinrichtung;
- **Fig.1m**: eine Skizze zur Erläuterung einer weiteren zum Ansatz an den Nasenöffnungsbereich vorgesehenen Abgriffseinrichtung mit Blenden- oder Drosselfenstern;
- **Fig**.**1n**: eine Skizze zur Erläuterung eines Abgriffkonzeptes mit aktiver Spülluftzufuhr;
- **Fig. 2**: eine Skizze zur Illustration eines modular aufgebauten Erfassungsgerätes, mit einem Game-Boy kompatiblen Untermodul;
- **Fig.3**: eine Bildschirmansicht zur Illustration einer Dartsellungsmöglichkeit der erfindugsgemäß erhobenen Messdaten unter Verwendung eines Auswertungsgerätes in Forme eines Rechners, z.B. Notebooks oder auch Game-Boys;
- **Fig. 4a**: ein Diagramm zur Erläuterung des Atemgasstromes für einen einzelnen Atemzug;
- **Fig. 4b**: ein Diagramm das den zeitlichen Verlauf des Atemgasstromes für mehrere Atemzüge beschreibt;
- **Fig. 4c**: ein Diagramm das den zeitlichen Verlauf des Atemgasdruckes mit einzelnen, durch Schnarchen verursachten Druckoszillationen darstellt;
- **Fig. 4d**: ein Diagramm das den zeitlichen Verlauf des Atemgasstromes für mehrere, durch eine Apnoe-Periode unterbrochene Atemzüge darstellt;
- **Fig. 5**: ein Diagramm das den zeitlichen Verlauf des Atemgasstromes mit einem Hypopnoeereignis darstellt;
- **Fig. 6**: ein Diagramm zum zeitlichen Verlauf des Atemgasstromes für mehrere, zum Teil flusslimitierte Atemzüge;
- **Fig. 7**: ein Diagramm zur Erläuterung des zeitlichen Verlaufs des Atemgasstromes im Falle einer im wesentlichen ungestörten, stabilen Atmung;
- **Fig. 8**: ein Diagramm zur Erläuterung des zeitlichen Verlaufes des Atemgasstromes im Falle einer instabilen, gestörten Atmung;
- **Fig. 9**: ein Diagramm das den zeitlichen Verlauf des Atemgasstromes darstellt wobei in dem Drucksignaloszillationen auftreten die durch Schnarchen verursacht sind;

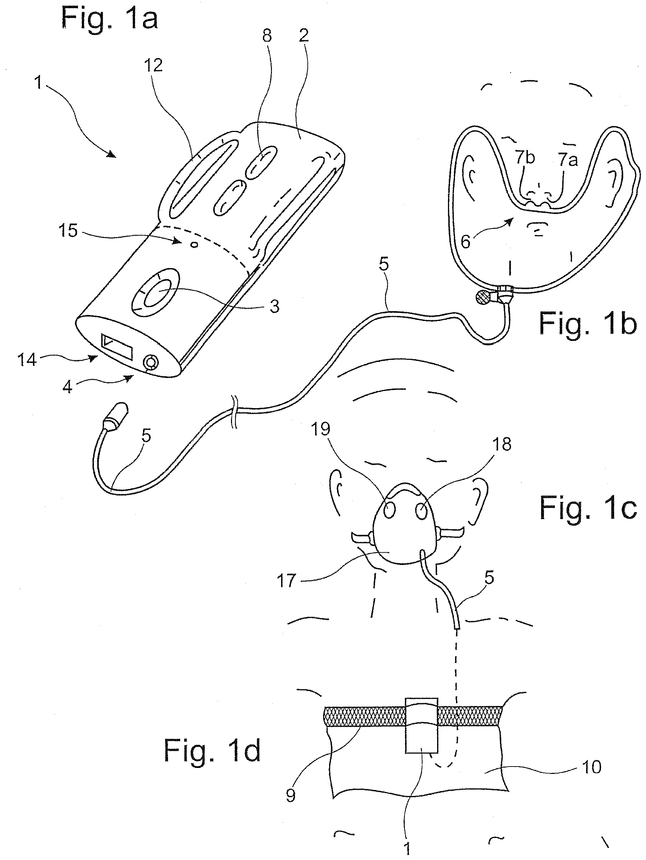

In Figur 1a ist ein mobiles Erfassungsgerät 1 dargestellt, das eine Gehäuseeinrichtung 2 und eine darin aufgenommene, hier nicht näher dargestellte Druck Sensoreinrichtung zur Erfassung und Generierung eines hinsichtlich des Atemgasstromes indikativen Atemstromsignals aufweist. Das Erfassungsgerät 1 umfasst ferner eine unter Einschluss einer Speichereinrichtung gebildete elektronischen Datenverarbeitungseinrichtung zur Verarbeitung des hinsichtlich des zeitlichen Verlaufs des Atemstrom indikativen Atemstromsignals. Die Datenverarbeitungseinrichtung ist derart konfiguriert, dass diese eine Speicherung von hinsichtlich des zeitlichen Verlaufs des Atemstromsignals indikativen Daten veranlasst.

Das gezeigte Erfassungsgerät 1 eignet sich insbesondere zur Anwendung im häuslichen Bereich, d.h. in der gewohnten Umgebung des Betroffenen zur Aufzeichnung von hinsichtlich der Atmung während einer Schlafphase charakteristischen Merkmale in einer Weise, die es gestattet, den physiologischen Zustand des Betroffenen mit hinreichend hoher Aussagekräftigkeit standardisiert zu bewerten. Auf Grundlage einer standardisierten Auswertung der erfassten Messergebnisse kann beurteilt werden, ob - und ggf. mit welchem Schweregrad das Krankheitsbild OSA vorliegt und ob eine tiefergehende Untersuchung durch ein Schlaflabor zu empfehlen ist.

Die Datenverarbeitungseinrichtung, ist derart konfiguriert, dass eine Prüfung erfolgt, ob das erfasste Atemstromsignal vorgegebene Signalqualitätskriterien erfüllt. Für den Fall, dass das anliegende Signal bestimmte Kriterien nicht erfüllt, wird die Signalaufzeichnung unterdrückt, und ein Dateieintrag veranlasst, aus welchem die zeitliche Lage von Perioden mit als unzulässig klassifizierten Signalen hervorgeht.

Die Datenverarbeitungseinrichtung ist derart ausgebildet, dass diese Zugriff zu einer Zeitgebereinrichtung hat, so dass die hinsichtlich des Atemstromsignals indikativen Daten mit einer Zeitinformation verknüpft aufgezeichnet werden können.

In Verbindung mit jener Datenverarbeitungseinrichtung ist ein Datenkompressionssystem verwirklicht, durch welches das erfasste, sich zeitlich ändernde Signal komprimiert aufgezeichnet wird.

Die Datenverarbeitungseinrichtung ist derart konfiguriert, dass der Aufzeichnungsvorgang durch einen anwenderseitig veranlassten Schaltimpuls ausgelöst wird. Die Einschaltung erfolgt indem der Schalttaster 3 über eine vorgegebene Mindestdauer von z.B. 3 Sekunden betätigt wird.

Die Datenverarbeitungseinrichtung ist derart ausgebildet, dass diese den Aufzeichnungsbeginn bzw. die Datenspeicherung veranlasst, wenn die anliegenden Atemstromsignale ein bestimmtes Kriterium z.B. ein vorgegebenes Periodizitätskriterium erfüllen.

Das Erfassungsgerät 1 weist einen ersten Druckmessanschluss 4 auf, an welchen eine Messkanüle 5 anschließbar ist.

Wie in Figur 1b dargestellt ist, kann diese Messkanüle 5 mit einer Nasaldruckmessbrilleneinrichtung 6 verbunden sein, zum Abgriff eines Staudruckereignisses aus dem, aus den Nasenöffnungen 7a, 7b abströmenden Atemgasstrom.

Es ist möglich, das Erfassungsgerät 1 mit einem zweiten Druckmessanschluss auszustatten, zur Erfassung eines zweiten Druckmesssignals. Durch die Möglichkeit der Erfassung von zwei Druckmesssignalen wird es möglich, das Erfassungsgerät 1 derart zu betreiben, dass dieses für die linke und rechte Nasenöffnung 7a, 7b jeweils indikative Atemstromsignale erfassen kann.

In Figur 1c ist eine weitere Variante zur Generierung eines hinsichtlich des Atemgasstromes indikativen Signals dargestellt. Diese Variante umfasst eine in der Art eines Mundschutzes ausgebildete Maske 17 aus einem gasdurchlässigen Material (z.B. Einweg-Papierfiltermatrerial). Durch diese Maske 17 wird es möglich, im Zusammenhang mit der Atmung im unmittelbaren Umgebungsbereich der Mund- und Nasenöffnung eine Druckdifferenz gegenüber der Umgebung zu erzeugen. Diese Druckdifferenz wird insbesondere durch die Dichtigkeit des Mundschutzes sowie die Durchlässigkeit des Masken- oder Filtermaterials bestimmt. Etwaige Nichtlinearitäten können im Bereich des Erfassungsgerätes ermittelt und kompensiert werden.

Der durch den als Diaphragma wirksamen Mundschutz definierte Maskeninnenraum steht über die Messkanüle 5 mit dem Erfassungsgerät in Verbindung. Es ist möglich, an der Maske Klappen- oder Ventileinrichtungen 18, 19 vorzusehen, durch welche der Einatmungsvorgang erleichtert wird. Über diese Ventil- oder Klappeneinrichtungen, insbesondere den Öffnungsgrad derselben ist es ebenfalls möglich, hinsichtlich der Atmung indikative Signale zu erfassen. Diese Signalübertragung kann drahtlos, insbesondere optisch, beispielsweise durch Infrarotlicht erfolgen.

Das hinsichtlich des Atemgasstromes indikative Signal kann weiterhin auch über anderweitige Messmittel, insbesondere Messblenden oder Volumenstromsensoren erfasst werden.

Das Erfassungsgerät 1 kann eine Einrichtung 8 zur Erfassung der Thoraxweitung aufweisen. Die Einrichtung zur Erfassung der Thoraxweitung kann wie in Figur 1d dargestellt ist ein Bandelement 9 umfassen, das um den Thoraxbereich des Anwenders 10 herumführbar ist. Es ist möglich, dieses Bandelement 9 derart auszugestalten, dass aus diesem ein hinsichtlich der Banddehnung, oder Bandbelastung indikatives Signal ableitbar ist. Es ist auch möglich, an dem Erfassungsgerät 1 Vorkehrungen zu treffen, durch welche die Bandbelastung erfasst werden kann. Insbesondere ist es möglich, an dem Erfassungsgerät 1 eine Schlaufeneinrichtung 12 vorzusehen, durch welche die Bandweitung, oder Bandkräfte erfasst werden können. Es ist auch möglich, an dem Erfassungsgerät 1 Druck- oder Krafterfassungsstrukturen 8 vorzusehen, durch welche die durch ein über diese Krafterfassungsstrukturen geführtes Band aufgebrachten Kräfte erfasst werden können. Das Band 9 zur Erfassung der Thoraxweitung kann zugleich der Befestigung des Erfassungsgeräts 1 am Anwender dienen. Es ist auch möglich, das Thoraxband lediglich zur Befestigung des Erfassungsgerätes 1 zu verwenden und von einer Erfassung des der Thoraxweitung abzusehen.

An dem Erfassungsgerät 1 können Elektrodeneinrichtungen vorgesehen sein durch welche EKG Signale vom Anwender abgegriffen werden können, indem das Erfassungsgerät unmittelbar auf die Haut des Anwenders aufgesetzt wird. Diese EKG-Signale können ebenfalls in zeitlicher Zuordnung aufgezeichnet werden.

Der Aufzeichnungsvorgang ist über den Schalttaster 3 anwenderseitig veranlassbar beendbar. Es ist weiterhin auch möglich, das Erfassungsgerät 1 derart zu konfigurieren, dass der Aufzeichnungsvorgang bei Erfüllung eines Zeitkriteriums beendet wird. Insbesondere ist es möglich, den Aufzeichnungsvorgang zu beenden, wenn dieser eine vorgegebene Aufzeichnungsdauer von z.B. 9,5 h erreicht hat.

Der Aufzeichnungsvorgang wird ferner auch schaltungsgesteuert beendet, wenn das anliegende Atemstromsignal über ein bestimmtes Abschaltzeitfenster ein bestimmtes Abschaltkriterium erfüllt.

Das Erfassungsgerät 1 ist mit einer Schnittstelleneinrichtung 14 versehen, zur Übertragung der aufgezeichneten Daten auf ein externes Auswertungssystem. Diese Schnittstelleneinrichtung ist hier als USB-Schnittstelle ausgeführt. Das Erfassungsgerät umfasst auch eine Infrarot-Schnittstelle zum potentialfreien Signalabgriff. Über diese Infrarotschnittstelle können während des Betriebs des Geräts die gewonnenen, hinsichtlich der Atmung generierten Signale zur anderweitigen Verwertung ausgelesen werden. Das Erfassungsgerät 1 kann insoweit als Messumformer betrieben werden.

Es ist auch möglich, das Erfassungsgerät 1 derart zu gestalten, dass die Speichereinrichtung austauschbar in dem Erfassungsgerät 1 aufgenommen, oder an diesem anbringbar ist. Die Speichereinrichtung kann hierzu als Karte oder insbesondere USB-Flashstick ausgebildet sein. Es ist möglich, durch einen vorab auf der Speichereinrichtung veranlassten Eintrag personenspezifische Daten auf dem Speichermedium aufzuzeichnen. Auf Grundlage dieser Vorab-Aufzeichnungen ist es möglich, eine Vorkonfiguration des Erfassungsgerätes 1 vorzunehmen, oder eine zutreffende Zuordnung der erfasten Daten zu dem Anwender sicherzustellen.

Die Konfiguration der Datenverarbeitungseinrichtung ist durch ein Datenverarbeitungsprogramm festgelegt, wobei dieses Datenverarbeitungsprogramm vorzugsweise veränderbar oder wechselbar ist. Die Abbildung des Datenverarbeitungsprogramms im Bereich des Erfassungsgerätes 1 kann über eine ROM-Einrichtung oder eine RAM-Einrichtung, insbesondere über die vorgenannten Schnittstelleneinrichtungen 14, zusätzliche Schnittstelleneinrichtungen, oder auch das Speichermedium erfolgen.

An dem Erfassungsgerät 1 sind neben einer vorzugsweise intuitiv leicht zu bedienenden Schaltereinrichtung 3 auch Anzeigeeinrichtungen 15 vorgesehen, zur Anzeige der Betriebsbereitschaft oder des Funktionszustands des Erfassungsgerätes. Bei der gezeigten Ausführungsform erfolgt die Anzeige der Aufzeichnungsbereitschaft des Erfassungsgerätes durch periodisches Aufblinken einer grünen Signaldiode.

Das Erfassungsgerät 1 umfasst eine Spannungsversorgungseinrichtung, die hier durch eine Batterieeinrichtung gebildet ist. Die Batterieeinrichtung ist kompakt ausgebildet, so dass das Erfassungsgerät flach und kleinbauend ausgeführt werden kann und nur ein geringes Eigengewicht aufweist.

Die Datenverarbeitungseinrichtung ist mit einer Kalibriereinrichtung gekoppelt, zur Kalibrierung des Atemstromsignals. Die Kalibriereinrichtung ist derart ausgebildet, dass diese eine selbsttätige Anpassung des Systems an die erfassten Signalpegel vornimmt.

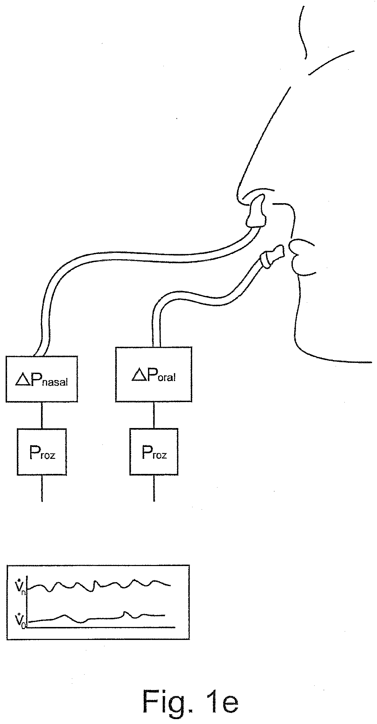

Figur 1e zeigt eine Skizze zur Illustration einer zweiten Ausführungsform eines erfindungsgemäßen Konzeptes zum separaten Abgriff eines hinsichtlich der Nasalatmung und eines hinsichtlich der oralen Atmung indikativen Signals. Diese Signale können als Drucksignale über Messkanülen erfasst werden. Die Signale können insbesondere als eine Druckdifferenz gegenüber dem Umgebungsdruck angebende Druckdifferenzsignale, gewonnen werden. die Signale können durch Signalverarbeitungsprozeduren normiert und überarbeitet werden. Die überarbeiteten Signale können zur Beschreibung, insbesondere Visualisierung des zeitlichen Verlaufs der Atmung verwendet werden.

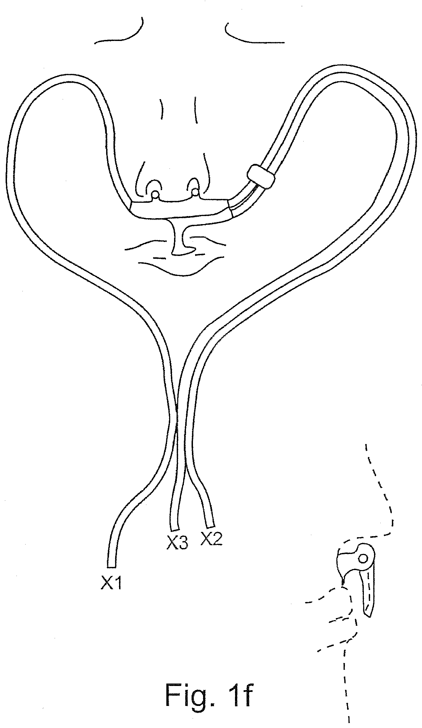

Fig.1f zeigt eine Skizze zur Erläuterung eines Konzeptes zur Überwachung der Atmung durch Abgriff von, für den Atemgasstrom durch die linke Nasenöffnung, die rechte Nasenöffnung sowie den Mund des Anwenders indikativen Signalen X1, X2, X3. Diese Signale können insbesondere über Druckmesskanülen abgegriffen werden. Die Signale X1, X2 können beispielsweise als Staudrucksignale durch eine Nasenbrillenanordnung erhoben werden. Das Signal X3 kann durch eine Messkanüle erfasst werden, die im Bereich der Oberlippe des Anwenders in einen Strömungsbereich eintaucht, über welchen ein Gasaustausch bei Mundatmung erfolgt. Es ist möglich, die Signale X1, X2, X3 zusammengefasst derart auszuwerten, dass das Summensignal ein Plausibilitätskriterium, beispielsweise im Hinblick auf das Atemzugsvolumen erfüllt.

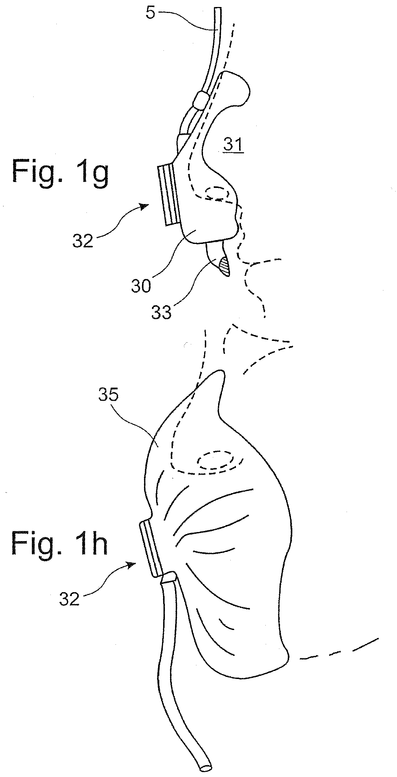

Fig.1g zeigt eine Skizze zur Erläuterung einer nasal zu applizierenden Abgriffseinrichtung mit integrierter Einrichtung zur Erfassung der Mundatmung. Die Abgriffseinrichtung umfasst einen Basiskörper 30, der aus einem Elastomermaterial, insbesondere Silikonkautschuk gefertigt ist. Der basiskörper definiert einen den Nasenspitzenbereich 31 eines Anwenders aufnehmenden und die Nasenöffnungen übergreifenden Abgriffsinnenraum. Dieser Abgriffsinnenraum steht über eine Messblendeneinrichtung 32 mit der Umgebung in Verbindung. Die Messblendeneinrichtung ist derart ausgebildet, dass diese im rahmen der Atemgasverlagerung zwischen dem Abgriffsinnenraum und der Umgebung einen relativ geringen, jedoch definierten Strömungswiderstand verursacht. Die infolge des Strömungswiderstandes der Messblendeneinrichtung im Abgriffsinnenraum gegenüber der Umgebung auftretenden Druckänderungen können über die Messkanüle 5 erfasst und zur Gewinnung hinsichtlich der nasalen Atmung indikative Daten konvertiert werden.

An dem Basiskörper 30 ist eine Sensoreinrichtung 33 vorgesehen, zum Abgriff eines hinsichtlich der Mundatmung indikativen Ereignisses, insbesondere Druckschwankung. Diese Druckschwankung kann ebenfalls durch eine Messkanüle oder anderweitige Signalübertragungseinrichtung einer weiteren Aufzeichnung zugeführt werden. Durch die Applikation einer zur Erfassung der oralen Atmung vorgesehenen Messeinrichtung über eine auf der Nase des Anwenders aufsitzende Struktur wird eine besonders vorteilhafte, insbesondere lagestabile und reproduzierbare Anordnung dieser Messeinrichtung gewährleistet.

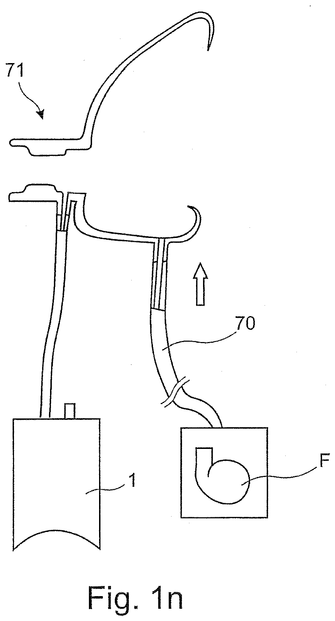

Die Messblende 32 kann als Gitter, Sieb, oder auch Gewebeelement ausgeführt sein. Wie später noch in Verbindung mit Figur 1n ausgeführt wird, ist es möglich, den Abgriffsinnenraum durch aktive Zufuhr eines atembaren Gases, insbesondere Umgebungsluf zu spülen. Hierdurch wird es möglich, auch bei besonders flacher Atmung einen ausreichenden Luftaustausch sicherzustellen. Weiterhin wird es möglich, das über die Kanüle 5 abgreifbare Drucksignal bei flacher Atmung noch in den positiven Bereich zu versetzen. Ein Unterdruck tritt dann erst auf, wenn der Inspirationsstrom größer ist als der Spülstrom.

Figur 1h zeigt eine Skizze zum Abgriff eines nasale und orale Atmungg zusammenfassenden Signals unter Verwendung einer die Nase und den Mundbereich übergreifenden Maskeneinrichtung 35. Die Maskeneinrichtung kann aus einem luftdurchlässigen Material gefertigt sein, oder wie hier gezeigt mit einer Drossel- oder Messblendeneinrichtung 32 versehen sein.

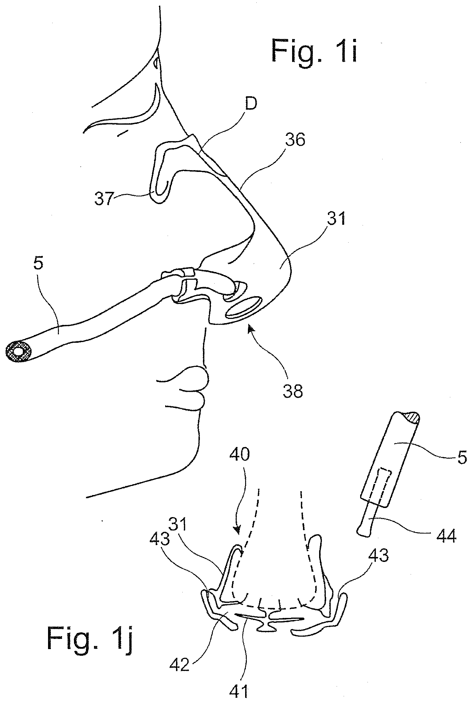

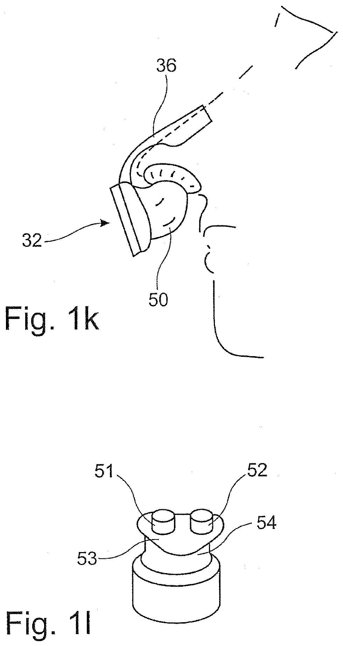

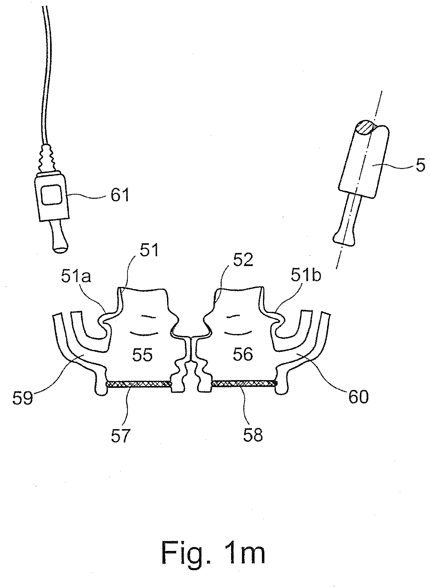

Fig.1i veranschaulicht eine Abgriffseinrichtung zum Abgriff eines hinsichtlich der nasalen Atmung indikativen Signales. Ähnlich wie die Variante nach Figur 1 umfasstt diese Abgriffseinrichtung einen Basiskörper 30 der einen die Nasenspitze aufnehmenden Abgriffsinnenraum definiert. Der Basiskörper 30 ist aus einem Kunststoffmaterial, vorzugsweise einem transparenten Elastomermaterial gefertigt.

Der Basiskörper 30 umfasst einen auf dem Nasenrücken verlaufenden Haltesteg 36. An dem Haltesteg 36 sind Halteflügel 37 ausgebildet. Der Haltesteg 36 und die Halteflügel 37 können über Klebebänder am Anwender fixiert werden, oder ggf, abschnittsweise selbstklebend ausgebildet sein. In dem Haltesteg 36 und/oder den Halteflügeln 37 können biegsame Einlagen, insbesondere Drahtabschnitte D verlaufen durch welche die Abgriffseinrichtung an die individuelle Nasentektur des Anwenders anpassbar ist.

Der Basiskörper 31 definiert einen Luftaustrittsabschnitt 38 über welchen eine Atemgasverlagerung von/zu den nasalen Atemwegen und der Umgebung erfolgen kann. Der Luftaustrittsabschnitt 38 kann so ausgebildet sein, dass dieser einen definierten Strömungswiderstand verursacht, so dass ein im Bereich des Abgriffsinnenraums herrschender Druck als hinsichtlich der Atmung indikatives Signal z.B. über die hier gezeigte Kanüle 5 abgegriffen werden kann.

Der Basiskörper 31 kann insbesondere in seinem, in Applikationsposition den Nasenöffnungen benachbartem Bereich so gestaltet sein, dass eine besonders vorteilhafte Erfassung der nasalen Atemgasströmung ermöglicht wird. Ein hierzu besonders geeigneter Aufbau ist Figur 1j skizziert.

Der Basiskörper 31 muss nicht zwingend aus einem Kunststoffmaterial gefertigt sein. Es ist auch möglich diesen aus einem Papier-, Zellstoff, Faser- oder anderweitigen insbesondere für Einweggebrauch geeigneten Material zu fertigen. Im Innenbereich des Basiskörpers kann abschnittsweise ein Schaumstoff- oder anderweitiges Polstermaterial vorgesehen sein, durch welches eine Abdichtung und Polsterung, insbesondere im Nasenrückenbereich erreicht wird.

Der in Figur 1j im Querschnitt vereinfacht dargestellte Basiskörper 31 ist mit einer Dichtlippenstruktur 40 versehen welche einen Abgriffsinnenbereich gegenüber der Umgebung abdichtet.

Der Abgriffsinnenbereich enthält eine Luftführungsstruktur die in dem die Nasenöffnungen umgebenden Bereich auf der Nase des Anwenders aufsitzt. Bei diesem Ausführungsbeispiel ist die Luftführungsstruktur so ausgebildet, dass diese eine hinsichtlich der linken und der rechten Nasalströmung separate Signalerhebung ermöglicht.

Die Luftführungsstruktur umfasst eine Umlenkplatte 41 über welche die über die Nasenöffnungen strömende Luft umgelenkt wird. In dem so geschaffenen Umlenkpfad ist an einer typischen Staudruckstelle ein Druckabgriffsport 42 vorgesehen. Der in diesem Druckabgriffsport herrschende Druck kann über eine Messkanüle 5 jeweils abgegriffen werden.

Der hier im Querschnitt gezeigte Basiskörper 31 ist aus einem Elastomermaterial gefertigt. Im Bereich des jeweiligen Druckabgriffsport sind elastische Einsteckkanäle 43 vorgesehen, in welche ein Einsteckstutzen 44 zur Ankoppelung der jeweiligen Kanüle 5 einsteckbar ist.

Der Umlenkpfad ist so ausgebildet, dass durch diesen eine Luftstromumlenkung um etwa 180° erfolgt. Der Druckabgriffsport 42 befindet sich jeweils im Bereich der Umlenkstelle.

Die Umlenkplatte 41 kann so ausgebildet sein, dass diese elastisch nachgiebig ist und bei höheren Atemgasströmen größere Durchgangsquerschnitte freigibt. es ist auch möglich, die Atemgasströmung anhand der Auslenkung der Umlenkplatte zu erfassen. Um ein Verstopfen des Druckabgriffports 42 zu vermeiden ist es möglich, die Messkanüle 5 permanent oder intermittierend mit einem Spülluftstrom zu beaufschlagen.

Figur 1k zeigt eine Abgriffseinrichtung mit jeweils in die Nasenöffnungen eingeführten und über einen auf dem Nasenrücken sitzenden Steg 36 fixierten Abgriffselementen 50. Die Abgriffselemente 50 sind aus einem elastomeren Material gefertigt und derart konturiert, dass diese vorteilhaft an den Nasenöffnungsbereich eines Anwenders ansetzbar sind. Die Abgriffselemente bilden Messkanalabschnitte über welche eine Erfassung des nasalen Atemgasstromes ermöglicht wird. Es ist möglich, diese Messkanalabschnitte an eine Messblende- oder Drosseleinrichtung 32 heranzuführen, über welche ein definierter Strömungswiderstand oder eine hinsichtlich der Signalerhebung vorteilhafte Strömungsbeeinflussung ermöglicht wird.

Figur 1l zeigt ein Abgriffselement mit zwei in die jeweilige Nasenöffnung einsetzbaren elastischen Stutzenabschnitten 51, 52, sowie einen eine axiale Abstützung ermöglichenden Bodenabschnitt 53. Beide Stutzenabschnitte 51, 52 münden bei diesem Ausführungsbeispiel in einen gemeinsamen Messkanalabschnitt 54 der über eine Messblende in die Umgebung mündet.

In Figur 1m ist in Form einer vereinfachten Querschnittsskizze eine weitere Variante einer Vorrichtung zum Abgriff hinsichtlich der nasalen Atemgasströmung indikativer Signale dargestellt. Bei diesem Ausführungsbeispiel sind die Stutzenabschnitte 51, 52 mit Balgstrukturen 51a, 51b versehen.

Die Abgriffseinrichtung bildet zwei Messkanäle 55, 56 die über Blenden- oder Drosselelemente 57, 58 mit der Umgebung in Verbindung stehen. Die Drosselelemente 57, 58 sind in eine Umfangsnut eingesetzt, die Mündungsbereich des jeweiligen Messkanals 55, 56 ausgebildet ist.

Der Abgriff des jeweiligen Drucksignals aus dem Bereich der Messkanäle erfolgt über Druckabgriffsportabschnitte 59, 60.

Die Signalerhebung kann wie vorangehend beschrieben über eine Kanüle 5 oder einen unmittelbar anschließbaren oder einsteckbaren Messumformer 61 erfolgen. Über den Messumformer 61 kann das hinsichtlich des Druckes in dem jeweiligen Messkanal indikative Signal in ein elektrisches, oder auch optisches Signal umgewandelt werden.

In Figur 1n ist skizzenhaft eine Messanordnung dargestellt, bei welcher der von einer Abgriffseinrichtung definierte Abgriffsinnenraum aktiv mit Spülluft beaufschlagt wird. Hierzu ist eine Spülluftleitung 70 vorgesehen, die in den Abgriffsinnenraum mündet. Die Spülluftzufuhr kann über eine Gebläseeinrichtung oder vorzugsweise über eine statische Fördereinrichtung, z.B. Zahnradpumpe oder anderweitigen volumetrischen Fördereinrichtung F erfolgen.

Die zwischen dem Abgriffsinnenraum und der Umgebung erfolgende Luftverlagerung kann über einen Pneumotachographen 71 erfasst und zur weiteren Auswertung durch das Erfassungsgerät 1 aufgezeichnet werden. Der sich insoweit ergebende Offset des Atemgasflussignales kann über die Auswertungsprozedur berücksichtigt werden. Die Spülluftleitung 70 kann einen kleinen Querschnitt von beispielsweise 10 mm² aufweisen. das Spülvolumen kann sich im Beriech von 1 bis 5 l/min bewegen.

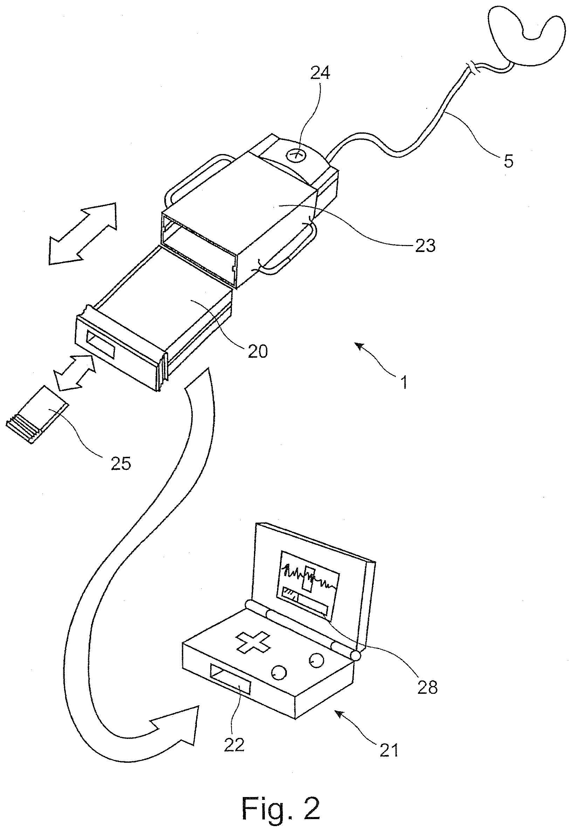

In Figur 2 ist eine Variante des Erfassungsgeräts 1 gezeigt, die eine Strukturkomponente 20 aufweist, die zu einem Wiedergabegerät 21 kompatibel ist, das in seinem Aufbau einem Game-Boy entspricht. Das Erfassungsgerät 1 ist damit derart gestaltet, dass zumindest ein Abschnitt desselben in einen Einschubschacht 22 eines Game-Boy einführbar ist.

Das Erfassungsgerät 1 ist derart ausgebildet, dass dieses ein Basismodul 23 und ein mit diesem koppelbares Aufzeichnungstransfermodul 20 umfasst.

Jenes Aufzeichnungstransfermodul 20 ist als Game-Boy kompatible Struktur ausgebildet. Hierdurch wird es möglich, die aufgezeichneten Daten über eine intuitiv leicht verständliche Benutzeroberfläche über ein handelsübliches Nutzerendgerät 21 zu visualisieren und ggf. im Hinblick auf ausgewählte Eigenschaften auszuwerten und zu verarbeiten. So ist es insbesondere möglich, ein zusammenfassendes Ergebnis in Form eines Schweregradbalkens 28 auszugeben. Durch dieses Balkendiagramm wird verdeutlicht, ob - und in welchem Ausmaß - eine behandlungsrelevante Störung vorliegt, oder nicht

Über das Basismodul 23 erfolgt vorzugsweise die Spannungsversorgung, sowie die Konvertierung des Drucksignales, über eine Kanüle 5 vom Anwender abgreifbar ist. Das Basismodul 23 umfasst hierzu einen Batterieschacht und einen Drucksensor sowie eine Schalteinrichtung 24.

Das Aufzeichnungsmodul 20 umfasst eine Datenverarbeitungseinrichtung die derart konfiguriert ist, dass diese eine Aufzeichnung von hinsichtlich des zeitlichen Verlaufs der Atmung indikativen Signalen auf einer Speichereinrichtung veranlasst. Das Aufzeichnungsmodul 20 kann mit einer Schnittstelleneinrichtung 14 versehen sein, zum Auslesen der aufgezeichneten Daten. Es ist möglich, die Speichereinrichtung 25 lösbar mit dem Aufzeichnungsmodul 20 zu koppeln, so dass es möglich wird, die Speichereinrichtung 25 von dem Aufzeichnungsmodul zu trennen und einem anderweitigen System zur weiteren Auswertung und Visualisierung zuzuführen.

Die Erfassung des Atmungssignals kann alternativ zu einer Erfassung unter Verwendung Nasalkanüleneinrichtung 5 auch über anderweitige Messmittel erfolgen.

Unter Verwendung des vorangehend genannten Erfassungsgerätes 1 wird es möglich ein hinsichtlich des physiologischen Zustands eines Anwenders indikatives Auswertungsresultats auf der Grundlage von Messsignalen die mit der Atmung einer Person im Zusammenhang stehen zu gewinnen, wobei aus den genannten Messsignalen unter Heranziehung standardisierter, Auswertungssysteme, Auswertungsmerkmale generiert werden und im Rahmen eines hierauf basierenden Resultat-Generierungsschrittes wenigstens ein Auswertungsresultat generiert werden kann, das den Schweregrad einer ggf. vorliegenden Erkrankung nach vorgegebenen Auswertungskriterien angibt, insbesondere visualisiert, z.B. in Form eines Balkendiagramms.

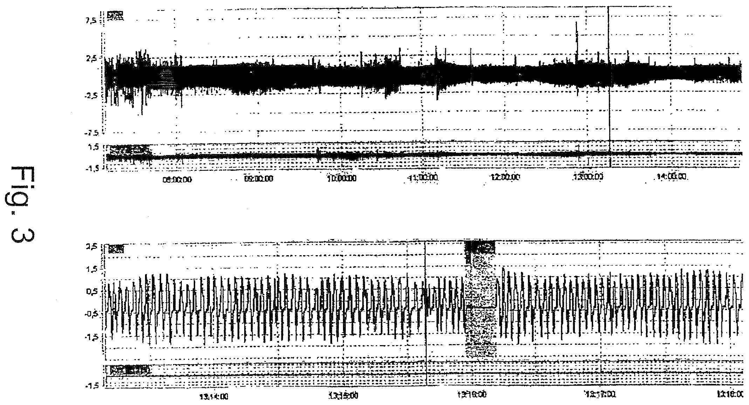

Die gesamten erfassten Daten können weiteren Auswertungsprozeduren zugeführt und wie beispielhaft in Figur 3 dargestellt unter einer komfortablen Menueoberfläche, graphisch visualisiert werden.

Durch das Erfassungsgerät und das mit diesem durchführbare Signalverarbeitungsverfahren wird es auf vorteilhafte Weise möglich, im Rahmen einer ca. 6 bis 8 Stunden andauernden anwenderseitig ambulant durchgeführten Signalerhebung eine Datenmenge zu schaffen, aus welcher Auswertungsmerkmale generiert werden können, aus welchen mit hoher Aussagefähigkeit in einer standardisiert wiederholbaren Weise Auswertungsresultate gewonnen werden können, die in vorteilhafter Weise einer nachfolgenden Diagnose zugrundegelegt werden können und damit eine zu einer standardisierten Bewertung beitragen.

Weitere Einzelheiten insbesondere für die Klassifizierung und automatische Bewertung der Atmung, ergeben sich aus der nachfolgenden Beschreibung.

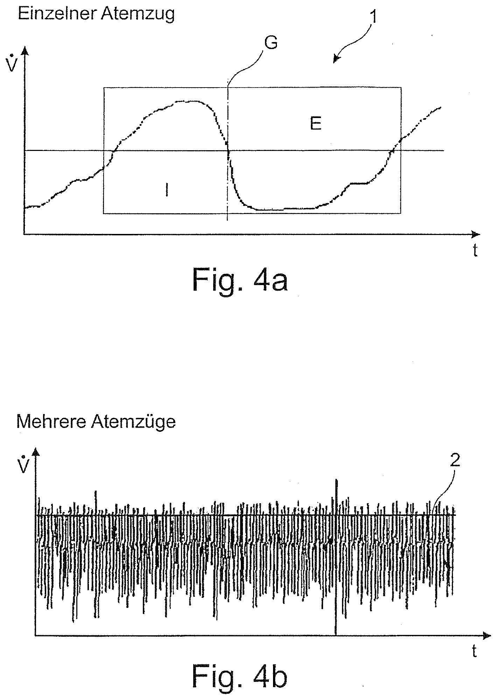

Der in Fig. 4a bezüglich des zeitlichen Verlaufs des Atemgasstromes dargestellte Atemzug 1 umfasst eine Inspirationsphase I und eine Expirationsphase E. Die Ermittlung der Atemphasengrenze G zwischen der Inspirationsphase und der Expirationsphase erfolgt durch überlagerte Auswertung mehrerer Kurvendiskussionskriterien, insbesondere auch unter Berücksichtigung des momentan vorherrschenden Atemmusters sowie der Extremwerte des Atemgasstromes und des Musters, des ermittelten Atemzugsvolumens sowie unter Berücksichtigung der Atemphasenperioden vorangegangener Atemzüge.

Der in Fig. 4a dargestellte Verlauf des Atemgasstromes beschreibt den Atemgasstromverlauf bei einem ungestörten Atemzug. Die Atemzugsbewertung kann anhand der zeitlichen Verhältnissen, z.B. der Inspirations- und Exspirationzeit zueinander oder zu anderen Eigenschaften z.B. der Gesamtatemzugslänge erfolgen. Gemäß einer besonders bevorzugten Ausführungsform der Erfindung wird der Quotient aus der Inspirationszeit und der Gesamtatemzugslänge berechnet um Veränderungen in der Atmung zu erkennen. In Fig. 4b ist der Verlauf des Atemgasstromes über ein längeres Zeitfenster hinweg dargestellt. Wie aus dieser Darstellung erkennbar ist, variieren die einzelnen Atemzüge insbesondere hinsichtlich in der hierbei auftretenden Minimas/Maximas. Die in dieser Darstellung eingetragene Horizontallinie 2 verdeutlicht den statistisch gesehen für Inspirationsphasen mit der höchsten Wahrscheinlichkeit auftretenden maximalen Atemgasstrom. Zusätzlich kann eine statistische Analyse der Inspiration- Exspirations- und Gesamtatemzugszeit über mehrere Atemzüge (vorzugsweise 10 Atemzüge) erfolgen.

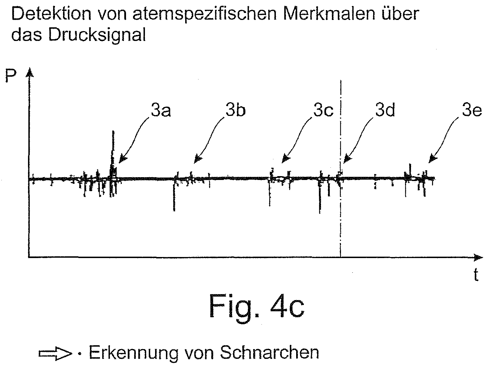

In Fig. 4c ist der zeitliche Verlauf eines hinsichtlich des Atemgasdruckes indikativen Signales dargestellt, wobei dieses Signal Oszillationssequezen 3a, 3b, 3c, 3d und 3e aufweist die durch Schnarchen verursacht sind. Die durch Schnarchen verursachten Druckschwankungen können über eine Anwendernahe Druckerfassungseinrichtung beispielsweise einen Atemgas-Druckmessschlauch erfasst werden. Es ist auch möglich, derartige Druckschwankungen über Mikrophoneinrichtungen zu erfassen.

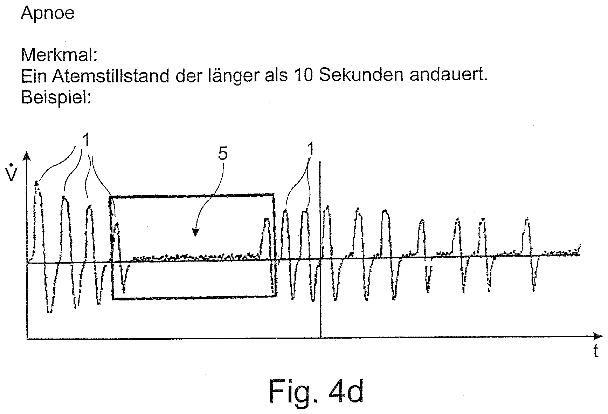

In Fig. 4d ist der zeitliche Verlauf des Atemgasstromes für mehrere, durch eine Atemstillstandsperiode 5 unterbrochene Atemzüge 1 dargestellt. Die auf Grundlage des Atemgasstromes erfasste Atemstillstandsperiode 5 weist eine, einen vorgegebenen Grenzwert von beispielsweise 10 Sekunden überschreitende Zeitdauer auf und wird damit als Apnoe-Phase klassifiziert. Sowohl die in dieser Darstellung vor der Atemstillstandsperiode 5 erfassten Atemzüge sowie die nachfolgenden Atemzüge zeigen Flusslimitationsmerkmale die in Zuordnung zu jedem Atemzug aufgezeichnet werden.

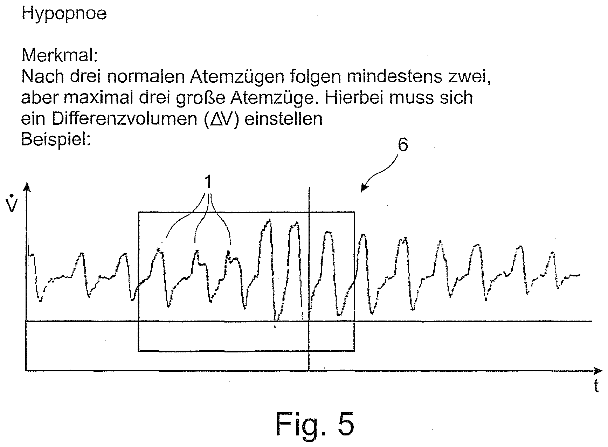

Fig. 5 zeigt den zeitlichen Verlauf des Atemgasstromes mit einer darin enthaltenen Hypopnoephase 6. Die Hypopnoephase 6 wird dann als vorliegend angesehen, wenn nach drei als normal eingestuften Atemzügen 1 mindestens zwei jedoch maximal drei Atemzüge folgen deren Differenzvolumen gegenüber den drei vorangegangenen Atemzügen einen vorgegebenen Grenzwert überschreitet.

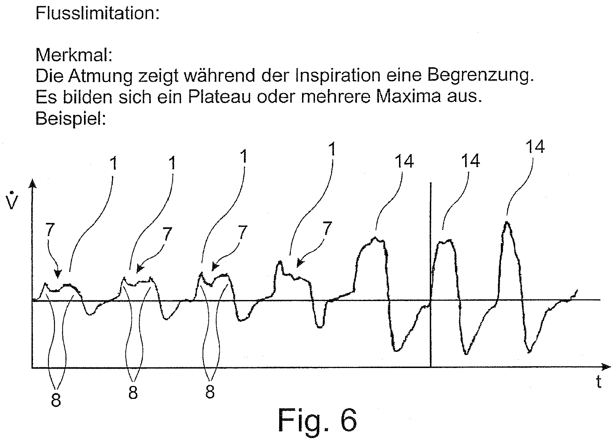

Fig. 6 zeigt den zeitlichen Verlauf des Atemgasstromes für mehrere Atemzüge, wobei die hier sichtbaren ersten 4 Atemzüge 1 Flusslimitationsmerkmale zeigen. Diese Flusslimitationsmerkmale sind in dem dargestellten Verlauf des Atemgasstromes durch darin ausgebildete Plateaus 7, sowie durch mehrere lokale Maxima 8 erkennbar. Bei den dargestellten Atemzügen treten die Flusslimitationsmerkmale jeweils in der Inspirationsphase des jeweiligen Atemzuges 1 auf. An die hier dargestellten ersten 4 Atemzüge 1 schließen sich drei zum Teil noch flusslimitierte Atemzüge 1 h an die einer Hypopnoephase zuzuordnen sind und zum Teil ebenfalls noch Flusslimitationsmerkmale zeigen.

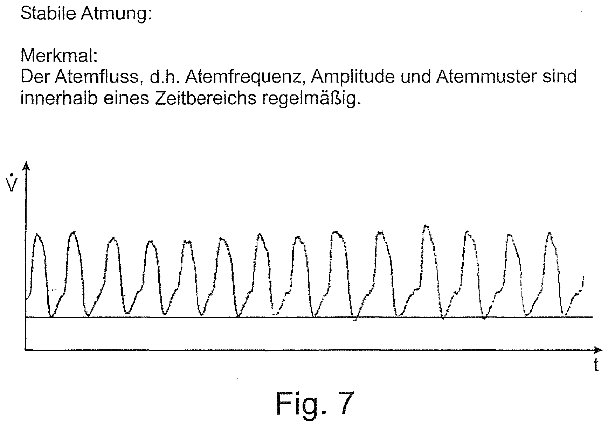

Fig. 7 zeigt den Verlauf des Atemgasstromes bei einer als stabil klassifizierten Atmungsperiode. Der Atemgasfluss, die Atemfrequenz, die Amplitude und das Atemmuster des Atemgasstromes sind innerhalb eines vorgegebenen Bereiches der als Zeitbereich oder auch durch eine Anzahl von Atemzügen definiert werden kann, regelmäßig. Die Atmungsstabilität bewegt sich bei dem hierbei dargestellten Verlauf des Atemgasstromes oberhalb eines Atmungsstabilitätsgrenzwertes von 0,86. Zusätzlich kann eine statistische Analyse der lnspirationszeit /Exspirationszeit und Gesamtatemzugszeit über mehrere Atemzüge (vorzugsweise 10 Atemzüge) erfolgen. Während der hier dargestellten Phase stabiler Atmung treten keine Atemstörungen (OSA) auf.

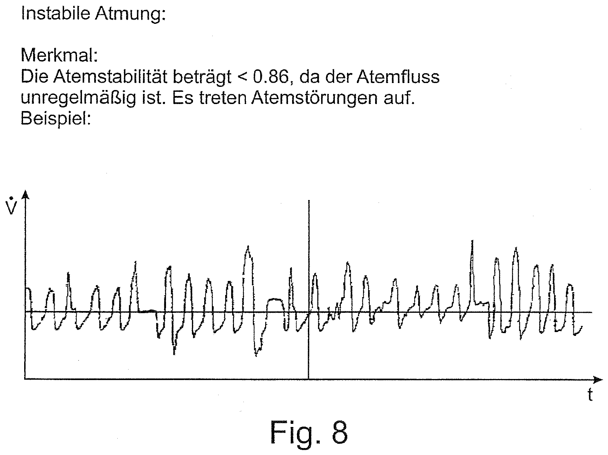

In Fig. 8 ist der zeitliche Verlauf des Atemgasstromes für mehrere Atemzüge dargestellt, wobei während des dargestellten Zeitabschnitts der Atemfluss unregelmäßig ist und bei einzelnen Atemzügen Atemstörungen (OSA) auftreten. Zusätzlich kann eine statistische Analyse der Inspirationszeit /Exspirationszeit und Gesamtatemzugszeit über mehrere Atemzüge (vorzugsweise 10 Atemzüge) erfolgen. Bei dem hier gezeigten Ausführungsbeispiel liegt der Atmungsstabilitätsindex unter dem Grenzwert von vorzugsweise 0,911.

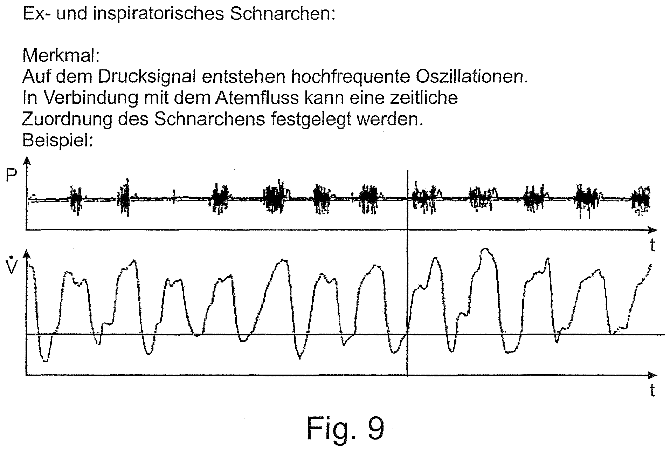

In Fig. 9 ist der zeitliche Verlauf des Atemgasstromes in Verbindung mit einem Atemgas-Drucksignal dargestellt. In dem Atemgas-Drucksignal sind phasenweise hochfrequente Oszilllationen enthalten die beim vorliegenden Beispiel inspiratorischem Schnarchen zugeordnet werden können.

## Patentansprüche

1. Verfahren zur Bereitstellung eines hinsichtlich des physiologischen Zustands indikativen Auswertungsresultats auf der Grundlage von Messsignalen, die mit der Atmung einer Person im Zusammenhang stehen,
wobei aus den genannten Messsignalen unter Heranziehung mehrerer Auswertungssysteme Auswertungsmerkmale generiert werden und im Rahmen eines hierauf basierenden Resultat-Generierungsschrittes wenigstens ein Auswertungsresultat generiert wird, indem die Auswertungsmerkmale einer verknüpfenden Betrachtung unterzogen werden,
wobei die Messsignale im Rahmen einer der Auswertung vorangehenden Signalerfassungsphase durch ein ambulantes Erfassungsgerät erfasst werden,
wobei die Messsignale, die mit der Atmung eines Person in Zusammenhang stehen, Atemstromsignale umfassen, und
wobei eine Datenverarbeitungseinrichtung einen Aufzeichnungsbeginn und/oder eine Datenspeicherung veranlasst, wenn die Atemstromsignale ein vorgegebenes Kriterium erfüllen.

2. Verfahren nach Anspruch 1, **dadurch gekennzeichnet, dass** das hinsichtlich des physiologischen Zustands indikative Auswertungsresultat mindestens Indikatoren der folgenden Indikatoren umfasst: Apnoe-Indikatoren, Hypopnoe-Indikatoren und Flusslimitationsindikatoren.

3. Verfahren nach wenigstens einem der vorangehenden Ansprüche, **dadurch gekennzeichnet, dass** ein überwiegend in den Auswertungsmerkmalen enthaltener Merkmalsbeitrag innerhalb eines Generierungs-Zeitfensters generiert wird, das kleiner ist als ein für die verknüpfende Betrachtung vorgesehenes Verknüpfungszeitfenster.

4. Verfahren nach wenigstens einem der vorangehenden Ansprüche, **dadurch gekennzeichnet, dass** auf Grundlage der verknüpfenden Betrachtung eine physiologische Typisierung des Anwenders zu obstruktiven, zentralen und/oder gemischten Atemstörungen erfolgt.

5. Verfahren nach wenigstens einem der vorangehenden Ansprüche, **dadurch gekennzeichnet, dass** die Auswertungsmerkmale auf Grundlage von Atemzugsstabilitätskriterien, auf Grundlage statistischer Auswertungsprozeduren und/oder als Merkmalsfeld generiert werden.

6. Verfahren nach wenigstens einem der vorangehenden Ansprüche, **dadurch gekennzeichnet, dass** als Auswertungsmerkmal mindestens eines der Folgenden generiert werden:
Normalatmungsphasenlängen, normalatmungscharakteristische Merkmale, Merkmale für regelmässige oder unregelmässige Atmungsphasenlängen, regelmässige und/oder unregelmässige Merkmale charakteristischer Auswertungsmerkmale, Flusslimitationsphasenlängen, flusslimitationscharakteristische Merkmale oder Datensätze, Merkmale für obstruktive Atmungsstörungen und/oder obstruktions-charakteristische Merkmale, Apnoe-Phasenlängen, apnoecharakteristische Merkmale oder Datensätze, Schnarchphasenlängen, schnarchphasencharakteristische Merkmale, Merkmale, die indikativ sind für das Auftreten von zentralen und/oder gemischten Atemstörungen, oder für das Verhältnis des Anteils oder der Zeitdauer von zentralen zu gemischten oder zentralen zu obstruktiven Atemstörungen, Merkmale für Cheyne-Stokes-Phasenlängen oder Cheyne-Stoke-charakteristische Merkmale oder Datensätze, Merkmale für periodische Vorgänge, zum Beispiel die Phasenlänge periodischer Vorgänge, Merkmale für die Hypoventilationsphasenlängen oder hypoventilationscharakteristische Merkmale oder Datensätze, Merkmale für atemzugsspezifische Zeiten, zum Beispiel Merkmale für die Inspiratioszeit, die Exspirationszeit und des Gesamtzyklus, Merkmale für den maximalen Atemvolumenstrom der Inspiration und Exspiration, mundatmungsindikative Merkmale der Inspiration und/oder der Exspiration, Lungenzugsvolumen-indikative Merkmale oder Datensätze, körperpositionsindikative Merkmale oder Datensätze, schlafphasencharakteristische Merkmale oder Datensätze.

7. Verfahren nach wenigstens einem der vorangehenden Ansprüche, **dadurch gekennzeichnet, dass** die generierten Auswertungsmerkmale unter Zuordnung ihrer zeitlichen Lage in dem Messsignalerhebungszeitraum gespeichert werden.

8. Verfahren nach wenigstens einem der vorangehenden Ansprüche, **dadurch gekennzeichnet, dass** im Rahmen der verknüpfenden Betrachtung mindestens eines der Folgenden generiert wird: ein Flusslimitationsindex, ein Apnoe/Hypopnoeindex, ein Schnarch-Index, ein Mundatmungs/Nasenatmungsindex, ein Schlafzeitindex, ein Schlafphasenindex, ein periodischer Atmungsindex, ein Atmungsvolumenindex.

9. Verfahren nach wenigstens einem der vorangehenden Ansprüche, **dadurch gekennzeichnet, dass** im Rahmen der verknüpfenden Betrachtung die Auswertungsmerkmale mit einer für die jeweilige Verknüpfung bestimmten Gewichtung berücksichtigt werden.

10. Verfahren nach wenigstens einem der vorangehenden Ansprüche, **dadurch gekennzeichnet, dass** zumindest ein Teil der Auswertungsmerkmale unter Berücksichtigung der ersten und/oder der zweiten Ableitung des zeitlichen Verlauf des Atemgasstromes generiert wird.

11. Verfahren nach wenigstens einem der vorangehenden Ansprüche, **dadurch gekennzeichnet, dass** durch das Messverfahren Beurteilungsgrundlagen für eine Prognose atembezogener Erkrankungen bereitgestellt werden.

12. Verfahren nach einem der vorangehenden Ansprüche, wobei das vorgegebene Kriterium ein vorgegebenes Periodizitätskriterium ist.

## Claims

1. A method for the provision of an evaluation result that is indicative of the physiological state on the basis of measurement signals associated with the respiration of a person,
wherein evaluation characteristics are generated from said measurement signals through the use of several analysis systems, and, in the course of a result generation step based thereon, at least one evaluation result is generated by subjecting the evaluation characteristics to an associative analysis,
wherein the measurement signals are acquired by a mobile detection appliance in the course of a signal acquisition phase preceding the evaluation,
wherein the measurement signals, which are associated with the breathing of a person, comprise respiratory flow signals; and
wherein a data processing device initiates a start of recording and/or a data storage when the respiratory flow signals fulfill a predefined criterion.

2. The method according to Claim 1, **characterized in that** the evaluation result indicative of the physiological state comprises at least indicators of the following indicators: apnea indicators, hypopnea indicators, and flow-limitation indicators.

3. The method according to at least one of the preceding claims, **characterized in that** a contribution to features predominantly contained in the evaluation characteristics is generated within a generation time window, which is smaller than a time window provided for the associative analysis.

4. The method according to at least one of the preceding claims, **characterized in that** a physiological classification of the user toward obstructive, central, and/or mixed respiratory disorders is carried out on the basis of the associative analysis.

5. The method according to at least one of the preceding claims, **characterized in that** the evaluation characteristics are generated on the basis of breathing stability criteria, on the basis of statistical analysis procedures, and/or as a characteristic field.

6. The method according to at least one of the preceding claims, **characterized in that** at least one of the following is generated as an evaluation characteristic:
normal respiratory phase lengths, normal respiratory characteristic properties, characteristics for regular or irregular respiratory phase lengths, regular and/or irregular characteristics of characteristic evaluation characteristics, flow limitation phase lengths, flow limitation characteristic characteristics or data sets, characteristics for obstructive respiratory disorders and/or obstruction characteristic characteristics, apnea phase lengths, apnea characteristic characteristics or data sets, snoring phase lengths, snoring phase characteristic characteristics, characteristics which are indicative for the occurrence of central and/or mixed respiratory disorders or for the ratio of the proportion or the time duration of central to mixed respiratory or central to obstructive respiratory disorders, characteristics for Cheyne-Stokes phase lengths or Cheyne-Stokes characteristic properties or data sets, characteristics for periodic events, for example the phase length of periodic events, characteristics for hypoventilation phase lengths or hypoventilation characteristic characteristics or data sets, characteristics for breath-specific times, for example characteristics for inspiratory time, expiratory time, and the entire cycle, characteristics for the maximum tidal volume flow for inspiration and expiration, characteristics indicative of oral respiration for inspiration and/or expiration, characteristics or data sets indicative of deep breathing volume, characteristics or data sets indicative of body position, characteristics or data sets characteristic of sleep phase.

7. The method according to at least one of the preceding claims, **characterized in that** the generated evaluation characteristics are stored by assigning their temporal location into the measurement signal collection time period.

8. The method according to at least one of the preceding claims, **characterized in that** at least one of the following is generated in the course of the associative analysis: a flow-limitation index, an apnea/hypopnea index, a snoring index, an oral respiration/nasal respiration index, a sleep time index, a sleep phase index, a periodic respiration index, a respiratory volume index.

9. The method according to at least one of the preceding claims, **characterized in that** the evaluation characteristics are considered with a certain weighting for the respective association in the course of the associative analysis.

10. The method according to at least one of the preceding claims, **characterized in that** at least one part of the evaluation characteristics is generated under consideration of the first and/or the second derivative of the time course of the respiratory gas flow.

11. The method according to at least one of the preceding claims, **characterized in that** the measurement method provides bases for assessment for a prognosis of breathing-related illnesses.

12. The method according to one of the preceding claims, wherein the predefined criterion is a predefined periodicity criterion.

## Revendications

1. Procédé pour fournir un résultat d'évaluation indicatif sur le plan de l'état physiologique sur la base de signaux de mesure qui sont en relation avec la respiration d'une personne,
dans lequel des caractéristiques d'évaluation sont générées à partir desdits signaux de mesure en exploitant plusieurs systèmes d'évaluation et au moins un résultat d'évaluation est généré dans le cadre d'une étape de génération de résultat basée sur celles-ci en soumettant les caractéristiques d'évaluation à une observation combinante,
dans lequel les signaux de mesure sont détectés par un dispositif de détection ambulant dans le cadre d'une phase de détection de signal précédant l'évaluation,
dans lequel les signaux de mesure qui sont en relation avec la respiration d'une personne comprennent des signaux de courant respiratoires, et
dans lequel un dispositif de traitement de données cause un début d'enregistrement et/ou un stockage de données lorsque les signaux de courant respiratoires remplissent un critère prédéfini.

2. Procédé selon la revendication 1, **caractérisé en ce que** le résultat d'évaluation indicatif sur le plan de l'état physiologique comprend au moins des indicateurs parmi les indicateurs suivants : indicateurs d'apnée, indicateurs d'hypopnée et indicateurs de limitation de flux.

3. Procédé selon au moins l'une des revendications précédentes, **caractérisé en ce qu'**une contribution de caractéristique contenue de manière prépondérante dans les caractéristiques d'évaluation est générée à l'intérieur d'une fenêtre de temps de génération qui est plus petite qu'une fenêtre de temps de combinaison prévue pour l'observation combinante.

4. Procédé selon au moins l'une des revendications précédentes, **caractérisé en ce qu'**un typage physiologique de l'utilisateur vis-à-vis de troubles respiratoires obstructifs, centraux et/ou mixtes a lieu sur la base de l'observation combinante.

5. Procédé selon au moins l'une des revendications précédentes, **caractérisé en ce que** les caractéristiques d'évaluation sont générées sur la base de critères de stabilité du souffle, sur la base de procédures d'évaluation statistiques et/ou comme champ de caractéristique.

6. Procédé selon au moins l'une des revendications précédentes, **caractérisé en ce qu'**au moins une des caractéristiques suivantes est générée comme caractéristique d'évaluation :
longueurs de phase d'une respiration normale, caractéristiques typiques d'une respiration normale, caractéristiques de longueurs de phase d'une respiration régulière ou irrégulière, caractéristiques régulières et/ou irrégulières de caractéristiques d'évaluation typiques, longueurs de phase d'une limitation de flux, caractéristiques ou jeux de données typiques d'une limitation de flux, caractéristiques de troubles respiratoires obstructifs et/ou caractéristiques typiques d'une obstruction, longueurs de phase d'une apnée, caractéristiques ou jeux de données typiques d'une apnée, longueurs de phase d'un ronflement, caractéristiques typiques de phases de ronflement, caractéristiques indicatives de la survenance de troubles respiratoires centraux et/ou mixtes, ou du rapport de la part ou de la durée de troubles respiratoires centraux par rapport à mixtes, ou centraux par rapport à obstructifs, caractéristiques de longueurs de phase de Cheyne-Stokes ou caractéristiques ou jeux de données typiques de Cheyne-Stokes, caractéristiques de processus périodiques, par exemple la longueur de phase de processus périodiques, caractéristiques des longueurs de phase d'une hypoventilation ou caractéristiques ou jeux de données typiques d'une hypoventilation, caractéristiques de périodes spécifiques du souffle, par exemple caractéristiques de la durée d'inspiration, de la durée d'expiration et du cycle complet, caractéristiques du débit respiratoire maximal de l'inspiration et de l'expiration, caractéristiques indicatives de la respiration par la bouche de l'inspiration et/ou de l'expiration, caractéristiques ou jeux de données indicatifs du volume d'inspiration, caractéristiques ou jeux de données indicatifs d'une position du corps, caractéristiques ou jeux de données typiques de phases du sommeil.

7. Procédé selon au moins l'une des revendications précédentes, **caractérisé en ce que** les caractéristiques d'évaluation générées sont enregistrées avec affectation de leur position temporelle dans la période de collecte des signaux de mesure.

8. Procédé selon au moins l'une des revendications précédentes, **caractérisé en ce qu'**au moins un des indices suivants est généré dans le cadre de l'observation combinante : un indice de limitation de flux, un indice d'apnée/d'hypopnée, un indice de ronflement, un indice de respiration par la bouche/indice de respiration par le nez, un indice de temps de sommeil, un indice de phase de sommeil, un indice de respiration périodique, un indice de volume respiratoire.

9. Procédé selon au moins l'une des revendications précédentes, **caractérisé en ce que**, dans le cadre de l'observation combinante sont prises en compte les caractéristiques d'évaluation avec une pondération déterminée pour la combinaison respective.

10. Procédé selon au moins l'une des revendications précédentes, **caractérisé en ce qu'**au moins une partie des caractéristiques d'évaluation est générée en prenant en compte la première et/ou la seconde dérivée de l'évolution dans le temps du débit de gaz respiratoire.

11. Procédé selon au moins l'une des revendications précédentes, **caractérisé en ce que** des bases d'estimation pour un pronostic de maladies liées à la respiration sont fournies par le procédé de mesure.

12. Procédé selon l'une des revendications précédentes, dans lequel le critère prédéterminé est un critère de périodicité prédéterminé.
